(19) Europäisches Patentamt
European Patent Office
Office européen des brevets

(11) Numéro de publication: 0 216 658 B1

(12) # FASCICULE DE BREVET EUROPEEN

(45) Date de publication du fascicule du brevet: 31.01.90

(51) Int. Cl.⁴: C 07 D 405/12, A 61 K 31/40

(21) Numéro de dépôt: 86401791.8

(22) Date de dépôt: 11.08.86

(54) **Nouveau benzodioxépanne, son procédé de synthèse et ses applications thérapeutiques.**

(30) Priorité: 12.08.85 FR 8512270

(43) Date de publication de la demande: 01.04.87 Bulletin 87/14

(45) Mention de la délivrance du brevet: 31.01.90 Bulletin 90/5

(84) Etats contractants désignés: AT BE CH DE FR GB IT LI LU NL SE

(56) Documents cités:
FR-A- 2 360 305
FR-A- 2 396 757
FR-A- 2 440 946

(73) Titulaire: SOCIETE D'ETUDES SCIENTIFIQUES ET INDUSTRIELLES DE L'ILE-DE-FRANCE, 46, Boulevard de Latour-Maubourg, F-75340 Paris Cédex 07 (FR)

(72) Inventeur: Besançon, Denis, Dr., 31 boulevard de Latour-Maubourg, F-75007 Paris (FR)
Inventeur: Franceschini, Jacqueline, 28 avenue Larroumès, F-94240 L'Hay-Les-Roses (FR)

EP 0 216 658 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

L'invention concerne le N-(1-cyclohéxènylméthyl 2-pyrrolidinyl méthyl) 8-éthylsulfonyl 1,5-benzodioxépanne 6-carboxamide, nouveau composé répondant è la formule suivante:

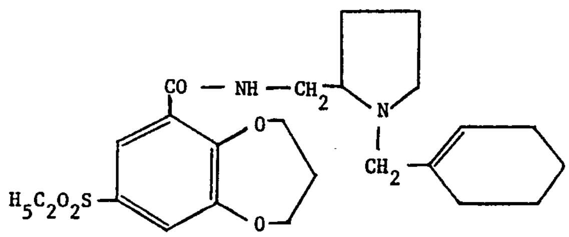

et ses sels pharmacologiquement acceptables.

D'après les demandes de brevets français publiées sous les n[os] Fr-A-2 360 305 et Fr-A-2 396 757, on connaissait des dérivés de N-(2-pyrrolidinylméthyl) 1,4-benzodioxanne 5-carboxamide et 1,5-benzodioxepanne 6-carboxamide, et en particulier des dérivés 7 (ou 8)-éthylsulfonyl, actifs comme psychostimulants, désinhibiteurs et thymoanaleptiques.

Le composé de l'invention, peut-être préparé par réaction de l'acide 8-éthylsulfonyl 1,5-benzodioxépanne 6-carboxylique avec le chlorure de thionyle, conduisant au chlorure d'acide correspondant, puis par réaction de ce chlorure de 8-éthylsulfonyl 1,5-benzodioxépanne 6-carbonyle avec la 1-(1-cyclohéxènylméthyl) 2-aminométhyl pyrrolidine.

L'acide 8-éthylsulfonyl 1,5-benzodixépanne 6-carboxylique de départ est lui-même préparé en traitant l'acide 1,5-benzodioxépanne 6-carboxylique par la chlorhydrine sulfurique, puis en transformant l'acide 8-chlorosulfonyl 1,5-benzodioxépanne 6-carboxylique obtenu (en deux étapes: réduction, puis éthylation) pour donner l'acide attendu.

L'exemple ci-après illustre la synthèse du composé objet de l'invention, selon le schéma réactionnel suivant:

I - *Acide 8-chlorosulfonyl 1,5-benzodioxépanne 6-carboxylique*

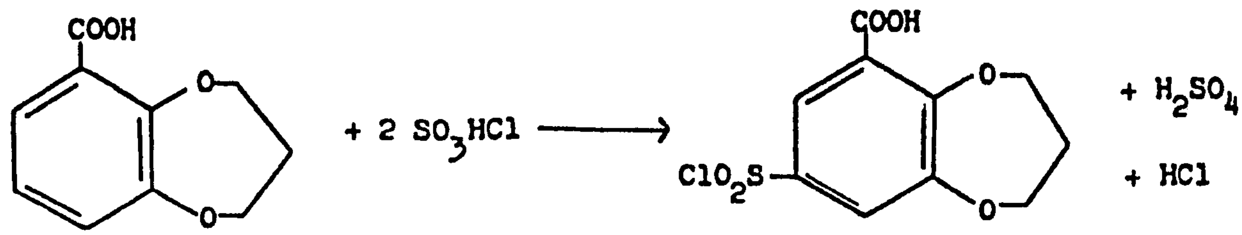


II - *Acide 8-éthylsulfonyl 1,5-benzodioxépanne 6-carboxylique*

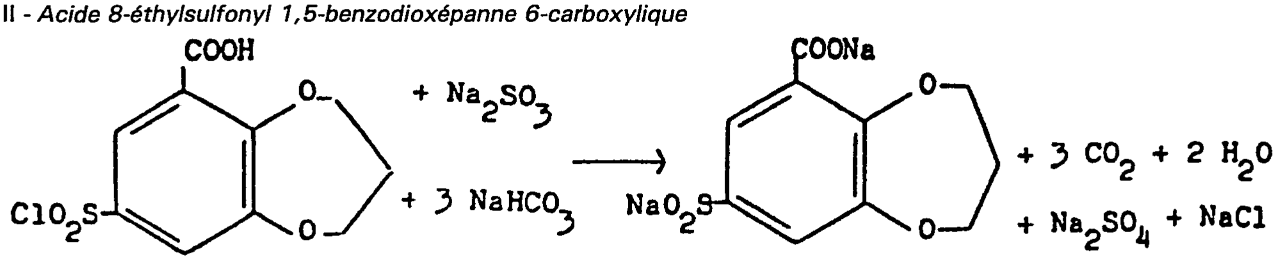


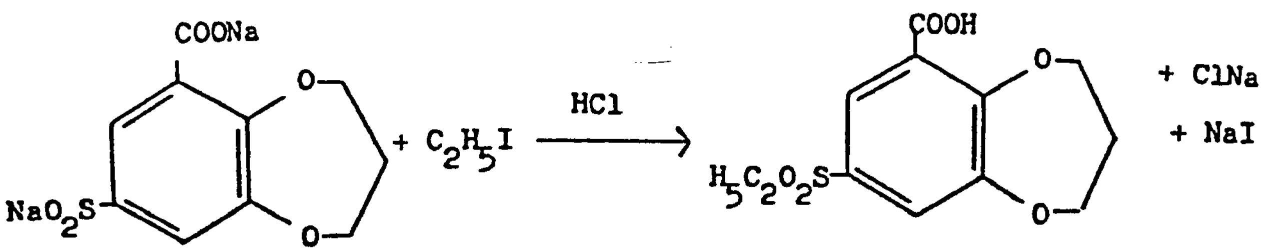


III - *Chlorure de 8-éthylsulfonyl 1,5-benzodioxépanne 6-carbonyle*

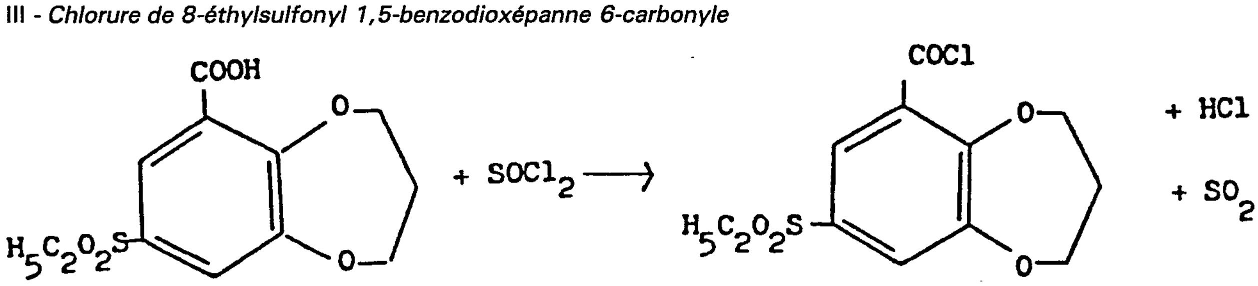

IV - *N(1-cyclohéxènylméthyl 2-pyrrolidylméthyl) 8-éthylsulfonyl 1,5-benzodioxépanne 6-carboxamide*

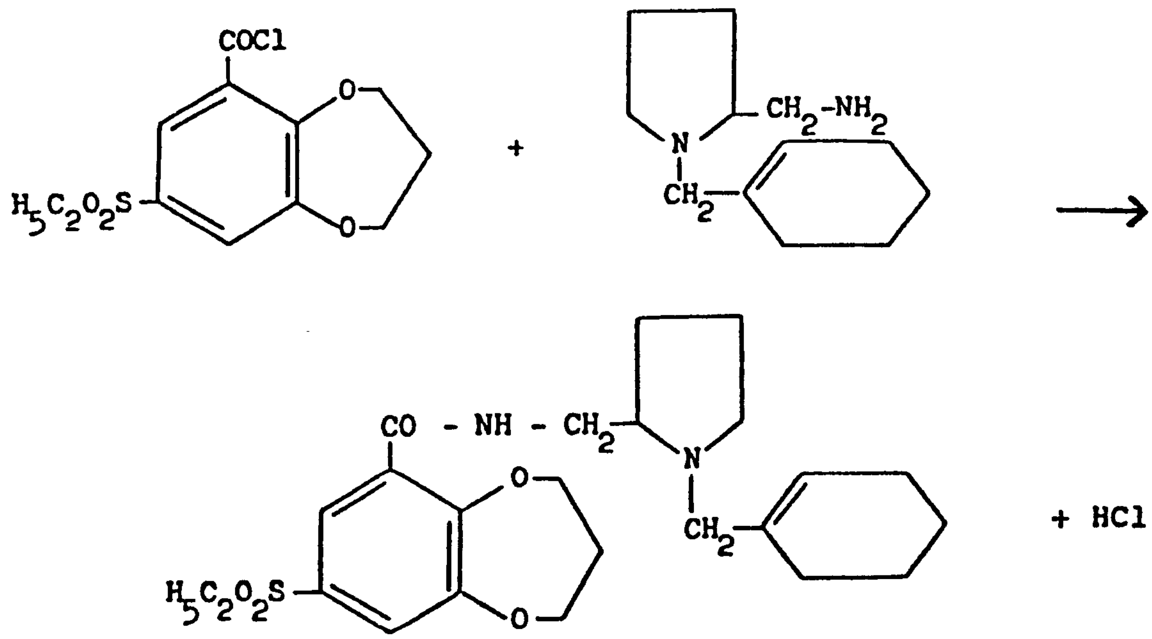

*Stade I*

*Acide 8-chlorosulfonyl 1,5-benzodioxépanne 6 carboxylique*

Dans un ballon de trois litres muni d'un agitateur étanche, d'un réfrigérant à reflux et d'un thermomètre, on introduit 1092 ml de chlorhydrine sulfurique (2 litres/mole), puis par portions, 106 g d'acide 1,5-benzodioxépanne 6-carboxylique (0,546 mole). La température est maintenue entre 5 et 10° par refroidissement extérieur. Chaque portion d'acide se dissout instantanément. L'introduction terminée, on laisse remonter la température et on agit encore pendant 5 heures. Puis on abandonne le mélange réactionnel une nuit à température ambiante. Dans un ballon de six litres muni d'un agitateur, d'un thermomètre et d'une ampoule à brome, on introduit 7,5 kg de glace et on coule goutte à goutte la solution de sulfochlorure. On refroidit extérieurement à l'aide de carboglace de manière à maintenir la température entre 0° et 5°. L'introduction dure 35 minutes. Le sulfochlorure cristallise immédiatement. Le produit obtenu est essoré, lavé à l'eau jusqu'à élimination des ions $Cl^-$ et séché à l'air.

Poids obtenu = 146 g. Rendement = 91%.

F = 114°-115°.

*Stade II*

*Acide 8-éthylsulfonyl 1,5-benzodioxépanne 6-carboxylique*

1 - *Réduction*

Dans un ballon de 10 litres muni d'un agitateur étanche, d'un réfrigérant à reflux à double enveloppe et d'un thermomètre, on introduit 1086 cc d'eau, 236 g de sulfite de sodium (1,25 mole + 50% d'excès) et 315 g de bicarbonate de sodium (1,25 mole × 3) et on chauffe à 65°-70°. On ajoute alors par portions 365 g d'acide 8-chlorosulfonyl 1,5-benzodioxépanne 6-carboxylique (1,25 mole). Il se produit un important dégagement de gas carbonique. L'introduction dure 2 heures environ. On continue de chauffer ensuite à 70°-80° jusqu'à fin de dégagement. On observe alors une perte de poids de 152 g pour 165 g en théorie.

2 - *Ethylation*

Après refroidissement, on ajoute 1296 cc d'éthanol, 585 g d'iodure d'éthyle (1,25 mole × 3) et 80 cc de lessive de soude à 30%, de manière à ce que le milieu soit alcalin à la phénolphtaléine, et on chauffe au reflux pendant 16 heures en rajoutant de la lessive de soude, dès que le milieu n'est plus alcalin, et en compensant les pertes d'iodure d'éthyle s'il y a lieu. En fin de réaction, on chauffe au reflux en milieu alcalin, pendant 4 heures. La réaction a duré au total 20 heures. On distille la majeure partie de l'alcool et reprend le résidu par 3 litres d'eau. On filtre la solution obtenue avec du noir puis l'acidifie par 400 cc d'acide chlorhydrique concentré. l'acide qui précipite est essoré, lavé à l'eau jusqu'à élimination des ions $CL^-$ et séché à 50°.

Poids obtenu = 311 g. Rendement = 84%.

F = 143°.

*Stade III*

*Chlorure de 8-éthylsulfonyl 1,5-benzodioxépanne 6-carbonyle*

Dans un ballon de 500 cc muni d'un réfrigérant à reflux, on introduit 200 g de chlorure de thionyle (0,42 mole × 4), puis la moitié environ de l'acide 8-éthylsulfonyl 1,5-benzodioxépanne 6-carboxylique total nécessaire (0,42 mole, soit 120 g) et on chauffe au bain d'eau à 40° jusqu'à ce que l'acide soit dissous en grande partie. On ajoute alors la deuxième moitié et on chauffe peu à peu jusqu'à

50°, puis au reflux jusqu'à dissolution totale. L'excès de chlorure de thionyle est alors distillé sous vide jusqu'à poids constant. Le chlorure d'acide restant cristallise.

Poid obtenu = 123 g. Rendement = 96%.

F = 125°.

*Stade IV*

*N(1-cyclohéxènylméthyl 2-pyrrolidylméthyl) 8-éthylsulfonyl 1,5-benzodioxépanne 6-carboxamide*

Dans un ballon de deux litres muni d'un agitateur, d'un thermomètre et d'une ampoule à brome, on introduit 78 g de 1(1-cyclohéxènylméthyl) 2-aminométhyl pyrrolidine (0,404 mole) et 600 cc de chloroforme et on ajoute peu à peu à la solution obtenue 123 g de chlorure de 8-éthylsulfonyl 1,5 benzodioxépanne 6-carboxyle (0,404 mole) finement pulvérisés, qui se dissolvent au fur et à mesure de l'addition. En fin d'introduction, le chlorhydrate formé cristallise. On maintient encore l'agitation 1 heure à 10°, puis on essore les cristaux, on les lave à la méthyléthyl-cétone puis à l'éther et on les sèche à l'air.

Poids obtenu = 144 g. Rendement = 71,5%.

Ces 144 g de chlorhydrate et 48 g de précédents essais, soit 192 g au total, sont dissous dans 800 cc d'eau. La solution obtenue est filtrée avec du noir, puis alcalinisée par 50 cc de lessive de soude. La base qui précipite sous forme pâteuse est extraite au chlorure de méthylène. La solution chlorométhylénique est séchée sur carbonate de potassium puis le chlorure de méthylène est distillé en terminant sous vide jusqu'à poids constant. Le résidu 173, est redissous dans 520 cc d'alcool à 95°. La cristallisation est amorcée. Elle se développe lentement. Au bout de 3 heures environ, les cristaux obtenus sont essorés, lavés à l'alcool à 95° puis à l'éther et séchés à l'air.

Poids obtenu = 119 g.

Rendement par rapport au chlorhydrate = 67%.

F = 103°.

*Caractéristiques du produit obtenu*

Cristaux blancs.

Soluble dans les acides dilués.

Très soluble à chaud dans le méthanol (2 volumes), très peu soluble à froid.

Très soluble à chaud dans l'acétate d'éthyle (2 volumes) soluble à froid dans 30 volumes.

F (Büchi) = 102°-104°.

Le composé de l'invention a fait l'objet de tests pharmacologiques qui ont donné les résultats suivants:

— Le composé se lie très fortement «in vitro» aux récepteurs dopaminergiques $D_2$.

En effet, on trouve à l'égard de cette classe de récepteurs, en utilisant le ligand $^3$H-spipérone qui les marque, une Cl 50 de $3,16 \times 10^{-9}$ M, alors que la liaison qux récepteurs D1 est faible (Cl 50 = 2,49 ou $6,31 \times 10^{-5}$ M) contre $^3$H-piflutixol. Cette forte liaison $D_2$ suggérait une action puissante sur ces récepteurs, la diffusion du produit à leur niveau paraissant également possible dans de bonnes conditions avec un coefficient de partage de 3,3.

— Le composé de l'invention est un puissant anti-dopaminergique au niveau du système nerveux central.

En effet, dans le test des stéréotypies provoquées par l'apomorphine, agoniste dopaminergique, chez le rat, le composé antagonise cette réponse comportementale à des doses très faibles. Ainsi quand les stéréotypies sont provoquées par 0,5 mg/kg d'apomorphine sous-cutanée, la dose inhibitrice 50 du composé est de 0,52 mg/kg par voie intrapéritonéale. Il est encore plus actif injecté par voie sous-cutanée: DI 50 = 0,039 mg/kg. Quand la dose d'apomorphine est supérieure, 1,25 mg/kg intraveineuse, le composé injecté par voie sous-cutanée exerce toujours un très puissant antagonisme, la DI 50 étant de 0,092 ou 0,082 mg/kg. De même, le composé antagonise puissamment les stéréotypies provoquées chez le rat par l'amphétamine, autre agoniste dopaminergique, à la dose de 10 mg/kg intraveineuse. Dans ce cas, la dose inhibitrice 50 du composé est également faible 0,062 mg/kg par voie sous-cutanée. Dans d'autres conditions expérimentales, l'amphétamine étant injectée par voie intrapéritonéale, la DI 50 du composé est de 0,4 mg/kg. D'après ces résultats, il apparait démontré que le composé s'oppose très efficacement aux effets centraux des agonistes dopaminergiques apomorphine et amphétamine et qu'en conséquence il possède à un très haut degré une propriété caractéristique des neuroleptiques. A cet égard, il se révèle nettement supérieur à un dérivé chimiquement apparenté (le N-(1-éthyl 2-pyrrolidinylméthyl) 8-éthylsulfonyl 1,5-benzodioxépanne 6-carboxamide (composé de référence), dont les doses inhibitrices 50 sont beaucoup plus élevées, démontrant une puissance d'effet neuroleptique très inférieure. Ainsi avec se composé, la DI 50 chez le rat est de 156 mg/kg par voie sous-cutanée contre 1,25 mg/kg d'apomorphine intraveineuse et de 36,4 mg/kg par voie intrapéritonéale contre 0,5 mg/kg d'apomorphine sous-cutanée, De même ce composé n'antagonise l'amphétamine qu'à dose élevée DI 50 = 39,4 mg/kg sous-cutanée.

— La propriété neuroleptique du composé est en outre démontrée par le fait qu'il exerce un effet cataleptigène, autre caractéristique des neuroleptiques. La dose cataleptigène 50 est de 5,3 mg/kg par voie sous-cutanée chez le rat. Un autre essai a donné une valeur un peu plus forte, 8,5 mg/kg sous-cutanée, également chez le rat. Par contre, le composé de référence est beaucoup moins cataleptigène, la DE 50 étant de 59,5 mg/kg par voie sous-cutanée, confirmant la moindre activité de ce composé par rapport à celui de l'invention.

— Le composé selon l'invention possède la propriété remarquable d'exercer rapidement son effet neuroleptique et d'autre part d'avoir un effet prolongé.

Ainsi, dans le test des stéréotypies à l'amphétamine chez le rat, l'action antagoniste avec la DI 50 rapportée ci-dessus (0,4 mg/kg sous-cutanée) se manifeste déjà à la 12e minute après l'injection sous-cutanée du composé. Dans un autre test les doses inhibitrices 50 du composé ont été évaluées, le

composé étant administré à des moments différents avant l'administration de l'agoniste, en l'espèce l'apomorphine à la dose de 0,5 mg/kg sous-cutanée. Il est ainsi possible de juger la puissance de l'antagonisme exercé par le composé à ces différents moments et d'apprécier aussi la durée de l'effet du composé. La DI 50 évaluée dans ces conditions était de 0,066 mg/kg sous-cutanée, le test étant réalisé 15 mn après l'injection. Ensuite, pour les délais de 30 à 360 mn les DI 50 sont comprises entre 0,035 et 0,049 mg/kg sous-cutanées, ce qui indique la permanence d'une action neuroleptique marquée. La cinétique de l'action du composé, indiquant l'établissement rapide de l'effet neuroleptique et sa longue durée, a été également mise en évidence par l'observation de l'effet cataleptigène. La catalepsie apparaît en effet 11 mn après l'injection sous-cutanée du composé et dure pendant plus de 5 h et jusqu'à 12 h chez le rat, selon les modalités de l'observation du phénomène.

— Comme on pouvait le prévoir avec un agent neuroleptique aussi puissant, le composé réduit la motilité spontanée chez la souris, qu'il soit administré par voie intrapéritonéale, les DI 50 étant alors de 0,58 à 0,93 mg/kg selon le protocole du test, ou par voie orale, les DI 50 étant alors de 13,2 à 16,4 mg/kg.

La cinétique de l'effet inhibiteur du composé a été étudiée chez la souris par l'évaluation des doses inhibitrices 50 à différentes distances de l'injection intrapéritonéale du composé. Après 5 mn, la DI 50 de 0,69 mg/kg démontre que l'effet inhibiteur se manifeste déjà puissamment. Ensuite, l'évolution des DI 50 entre 0,55 et 0,9 indique la persistance de l'effet jusqu'à 5 h. Ensuite, l'effet s'estompe, mais est encore observable plus de 5 h après l'administration, ce qui s'accorde avec la durée attribuée à l'effet cataleptigène.

— Le composé possède en outre une propriété remarquable et inattendue, à savoir qu'à des doses très faibles où son effet neuroleptique ne se manifeste pas encore, il exerce un certain effet activant se traduisant par une augmentation de l'activité motrice mesurée par un procédé photoélectrique. Cette activation intervient significativement à des doses comprises entre 0,0001 et 0,5 mg/kg souscutanée. D'autre part, aux doses supérieures neuroleptiques qui réduisent la motricité, les animaux traités par le composé conservent une certaine vigilance, gardant notamment les yeux ouverts, ce qui est inhabituel avec les neuroleptiques puissants.

Le composé de l'invention présente les caractéristiques pharmacologiques d'un puissant neuroleptique, agissant rapidement et de façon durable et pouvant en conséquence être appliqué dans les états d'agitation des psychoses aiguës et chroniques.

**Revendications pour les Etats contractants:**
BE, CH, DE, FR, GB, IT, LI, LU, NL, SE

1. Le N(1-cyclohéxènylméthyl 2-pyrrolidinylméthyl) 8-éthylsulfonyl 1,5-benzodioxépanne 6-carboxamide et ses sels physiologiquement acceptables.

2. Procédé de préparation du composé selon la revendication 1, qui consiste à traiter l'acide 1,5-benzodioxépanne 6-carboxylique par la chlorhydrine sulfurique pour obtenir l'acide 8-chlorosulfonyl 1,5-benzodioxépanne 6-carboxylique, à traiter ce dernier par le sulfite et le bicarbonate e sodium, puis par l'iodure d'éthyle, à traiter l'acide 8-éthylsulfonyl 1,5-benzodioxépanne 6-carboxylique obtenu par le chlorure de thionyle, et à amidifier le chlorure d'acide résultant par la 1(1-cyclohéxènylméthyl) 2-amino méthyl pyrrolidine.

3. Médicament caractérisé en ce qu'il comprend le composé selon la revendication 1.

4. Composition pharmaceutique contenant le composé selon la revendication 1, associé à un excipient pharmacologiquement acceptable.

**Revendication pour l'Etat contractant:** AT

Procédé de préparation du N-(1-cyclohéxènylméthyl 2-pyrrolidinylméthyl) 8-éthylsulfonyl 1,5-benzodioxépanne 6-carboxamide et de ses sels physiologiquement acceptables, qui consiste à traiter l'acide 1,5-benzodioxépanne 6-carboxylique par la chlorhydrine sulfurique, à traiter l'acide 8-chlorosulfonyl 1,5-benzodioxépanne 6-carboxylique ainsi obtenu par le sulfite et le bicarbonate de sodium puis par l'iodure d'éthyle, à traiter l'acide 8-éthylsulfonyl 1,5-benzodioxépanne 6-carboxylique obtenu par le chlorure de thionyle et à amidifier le chlorure d'acide résultant par la 1-(1-cyclohéxènylméthyl) 2-aminométhyl pyrrolidine.

**Patentansprüche für die benannten Vertragsstaaten:**
BE, CH, DE, FR, GB, IT, LI, LU, NL, SE

1. N-(1-Cyclohexenylmethyl-2-pyrrolidinylmethyl)-8-ethylsulfonyl-1,5-benzodioxepan-6-carboxamid sowie seine physiologisch verträglichen Salze.

2. Verfahren zur Herstellung der Verbindung nach Anspruch 1, dadurch gekennzeichnet, daß man 1,5-Benzodioxepan-6-carbonsäure mit Chlorschwefelsäure zur 8-Chlorosulfonyl-1,5-benzodioxepan-6-carbonsäure umsetzt, das erhaltene Produkt mit Natriumsulfit und -bicarbonat sowie anschließend mit Ethyljodid behandelt, die erhaltene 8-Ethylsulfonyl-1,5-benzodioxepan-6-carbonsäure mit Thionylchlorid umsetzt und das erhaltene Säurechlorid mit 1-(1-Cyclohexenylmethyl)-2-aminomethylpyrrolidin amidiert.

3. Arzneimittel, gekennzeichnet durch eine Gehalt an der Verbindung nach Anspruch 1.

4. Pharmazeutische Zusammensetzung, enthaltend die Verbindung nach Anspruch 1 zusammen mit einem pharmakologisch verträglichen Exzipienten.

**Patentanspruch für den benannten Vertragsstaat:** AT

Verfahren zur Herstellung von N-(1-Cyclohexenyl-methyl-2-pyrrolidinylmethyl)-8-ethylsulfonyl-1,5-benzodioxepan-6-carboxamid sowie seiner physiologisch verträglichen Salze, dadurch gekennzeichnet, daß man 1,5-Benzodioxepan-6-carbonsäure mit Chloroschwefelsäure behandelt, die so erhaltene 8-Chlorosulfonyl-1,5-benzodioxepan-6-carbonsäure mit Natriumsulfit und -bicarbonat sowie anschließend mit Ethyljodid behandelt, die erhaltene 8-Ethylsulfonyl-1,5-benzodioxepan-6-carbonsäure mit Thionylchlorid behandelt und das erhaltene Säurechlorid mit 1-(1-Cyclohexenylmethyl)-2-aminomethylpyrrolidin amidiert.

**Claims for Contracting States:**
BE, CH, DE, FR, GB, IT, LI, LU, NL, SE

1. N-(1-cyclohexenylmethyl-2-pyrrolodinylmethyl)-8-ethylsulphonyl-1,5-benzodioxepan-6-carboxamide and its physiologically acceptable salts.

2. Process for the preparation of the compound according to claim 1, consisting of treating 1,5-benzodioxepan-6-carboxylic acid with sulphuric hydrochlorin to obtain 8-chlorosulphonyl-1,5-benzodioxepan-6-carboxylic acid, treating the latter with sodium sulphide and biocarbonate and then ethyl iodide, treating the 8-ethylsulphonyl-1,5-benzodioxepan-6-carboxylic acid obtained with thionyl chloride and amidating the resulting acid chloride with 1-(1-cyclohexenylmethyl)-2-aminomethyl pyrrolidine.

3. Medicament characterized in that it comprises the compound according to claim 1.

4. Pharmaceutical composition containing the compound according to claim 1 associated with a pharmacologically acceptable excipient.

**Claims for the Contracting State:** AT

Process for preparing N-(1-cyclohexenylmethyl 2-pyrrolidinylmethyl) 8-ethylsulphonyl 1,5-benzodioxepan-6-carboxamide and physiologically acceptable salts thereof, consisting of treating 1,5-benzodioxepan 6-carboxylic acid with sulphuric chlorohydrin, treating the resulting 8-chlorosulphonyl 1,5-benzodioxepan-6-carboxylic acid with sodium sulphite and bicarbonate then with ethyl iodide, treating the resulting 8-ethylsulphonyl 1,5-benzodioxepan 6-carboxylic acid with thionyl chloride and finally amidating the resulting acid chloride with 1-(1-cyclohexenylmethyl) 2-aminomethyl pyrrolidine.